# EUROPEAN PATENT APPLICATION

(11) **EP 4 541 884 A1**
(43) Date of publication of application: **23.04.2025**
(21) Application number: 23203818.2
(22) Date of filing: 16.10.2023
(51) Int. Cl.: C12N 9/04, C12P 19/02

(54) **MODIFIED XYLOSE REDUCTASE AND USES THEREOF**

(71) Applicant: Annikki GmbH, 8074 Raaba-Grambach (AT)
(72) Inventor: Staunig, Nicole, 8074 Raaba-Grambach (AT); Dupont, Maria, 8074 Raaba-Grambach (AT); Koch, Dennis, 8074 Raaba-Grambach (AT)
(74) Representative: Schwarz & Partner Patentanwälte GmbH

(57) **Abstract**

The present invention relates to a method for producing D-fructose comprising the step of reducing D-glucosone to D-fructose with a modified xylose reductase enzyme or a functional fragment thereof, wherein the modified xylose reductase enzyme has a lower D-glucose specificity and a lower D-xylose specificity compared to the unmodified xylose reductase enzyme.

## Description

### TECHNICAL FIELD

The present invention relates to the field of enzymes, in particular enzymes capable to reduce D-glucosone to D-fructose.

### BACKGROUND ART

D-glucose is present in large quantities in various biopolymers like starch and cellulose that are part of renewable raw materials. D-glucose can be isomerized to D-fructose which plays an important role in sugar industry as it has higher sweetness than D-glucose, but can also be converted to 5-(hydroxymethyl)furfural (HMF), a precursor of 2,5-furandicarboxylic acid (FDCA). FDCA is used for the synthesis of furan polymers like poly(ethylene)furanoate (PEF) - a sustainable alternative to PET, which is based on fossil fuels (Milić et al., 2023).

D-fructose is also reduced to D-mannitol and D-sorbitol. Both sugar alcohols are low-calorie and non-cariogenic sweeteners. Furthermore, D-mannitol is finding growing use in food products for diabetic and/or obese patients, in pharmaceuticals (as a diuretic and in the treatment of renal failure, for tablet formulations, or as a drug carrier in dry powder inhalers), and for the production of specialty chemicals (Grembecka, 2015).

There are different ways to produce D-fructose from D-glucose. One method in steps has been used for production at bulk scale that is characterized by D-glucose formation via hydrolysis of polysaccharides and, afterwards, the isomerization of D-glucose into D-fructose which yields a mixture of 42% D-fructose, 50% D-glucose and 8% residual polysaccharides. A further accumulation of D-fructose is achieved by separation methods. It is possible to use chromatographic methods, e.g. described in US 5,221,478. Especially, the production of highly pure D-fructose using chromatographic methods is elaborate and very costly.

An alternative method is to generate D-fructose from D-glucose by combining an enzymatic and a chemical step. The D-fructose production in such way is disclosed in US 4,246,347 where 2% D-glucose is initially converted to D-glucosone using a py-ranose-2-oxidase. In a second step, D-glucosone is then converted into D-fructose by hydrogenation. For the hydrogenation to proceed high pressures and temperatures are required and reaction is conducted at low substrate concentration.

In addition to using a combination of an enzymatic and a chemical step, two-step processes combining enzymatic redox reactions on carbohydrates have also been described. US 10,253,340 discloses the xylose reductase (XR)-dependent production of D-fructose from D-glucose via D-sorbitol as intermediate. XR first generates D-sorbitol which is subsequently reduced by sorbitol dehydrogenase. Cofactor recycling can be implemented for each step.

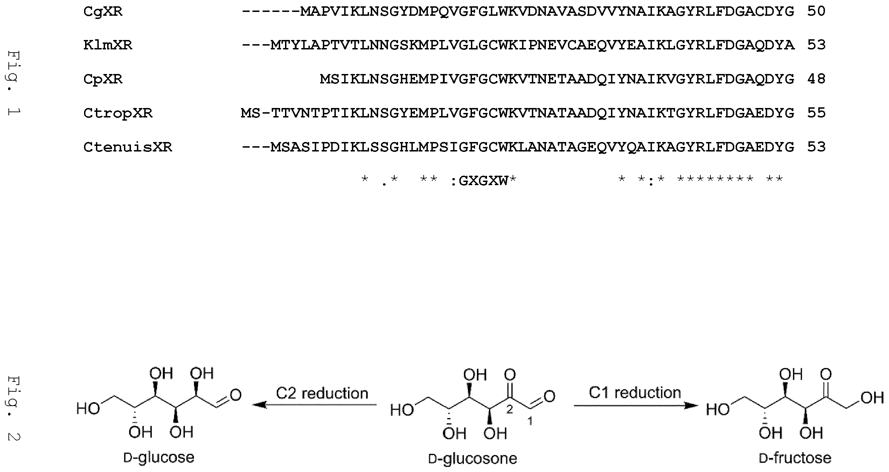

XRs are NAD(P)-dependent reductases (Liang et al., 2007) that have a plethora of different aldehyde-containing substrates, among them D-xylose, D-glucose or 2-keto-D-glucose (D-glucosone), but not sugars that contain a keto-group, such as D-fructose (Neuhauser et al., 1997). The tryptophan (W) residue in the GXGXW motif (defined in Fig. 1) leads to aldehyde reduction (C1-carbonyl group), whereas mutation of the W to Y or F results in a strong preference for ketone reduction (C2-carbonyl group) (Kratzer et al., 2006). As a result, the catalytic mechanism switches from C1-carbonyl reduction to C2-carbonyl reduction when W is mutated to Y or F.

XRs are for example implemented in production processes of deoxyketoses (US 5,077,203) and xylitol (CN 110628835). Also, redox methods that do not require xylose reductase activity are supplemented with external cofactor (CN 111876452) .

Known methods for D-fructose production have different drawbacks that render them unattractive. Chemocatalytic processes usually run at high pressures and temperatures, show low product yield and require laborious and cost-intensive separation.

Biocatalytic redox processes are widely used to replace more expensive chemical synthesis, as no extensive amounts of waste are formed, and the processes can be performed in shorter time using milder reaction conditions (ambient pressure and temperature) .

US 10,113,192 discloses the XR-dependent production of D-fructose from D-glucose via D-glucosone as intermediate. In a one-pot set up during which D-glucose is oxidized to D-glucosone as intermediate by a pyranose-2-oxidase, XR subsequently generates D-fructose using D-glucosone as substrate. A disadvantage of this enzymatic system is that the process is running at relative low substrate concentration (a substrate load of 2.5% D-glucose is converted to 91% D-fructose in 48 h). At higher substrate concentration a byproduct (D-sorbitol) accumulates in the reaction vessel to up to 5-10 %.

The rate of bioconversion of D-glucose increased with the pressure since an increase in the pressure with compressed air resulted in higher rates of conversion (Karmali & Coelho, 2011).

It is an object of the present invention to provide an efficient method to produce pure D-fructose from D-glucose with D-glucosone as intermediate at high substrate concentration by using modified XRs.

### SUMMARY OF THE INVENTION

Surprisingly, it was found that modified xylose reductase enzymes or functional fragments thereof exhibiting a lower D-glucose specificity and a lower D-xylose specificity compared to the respective unmodified xylose reductase enzyme can be used to produce D-fructose highly efficiently. The reduced D-glucose and D-xylose specificity helps to reduce or even abolish the formation of byproduct D-sorbitol by simultaneous enhancement of the D-glucosone activity. The reduction of the D-glucose and D-xylose specificity can be achieved by mutating the xylose reductase conserved consensus sequence. Compared to previous art (Kratzer et al., 2006), the inserted mutations did not affect the catalytic mechanism.

The "unmodified xylose reductase enzyme", as used herein, is able to reduce D-glucosone to D-fructose, however, the unmodified xylose reductase enzyme shows a higher D-glucose specificity and a higher D-xylose specificity compared to the respective modified xylose reductase enzyme.

The present invention relates also to the economical operation of xylose reductase (XR)-dependent redox processes at high substrate concentration by implementation of a xylose reductase following the disclosed consensus.

Another aspect of the present invention relates to a modified xylose reductase enzyme or a functional fragment thereof, wherein the modified xylose reductase enzyme has a lower D-glucose specificity and a lower D-xylose specificity compared to the unmodified xylose reductase enzyme.

A further aspect of the present invention relates to a nucleic acid molecule encoding a modified xylose reductase enzyme according to the present invention.

Another aspect of the present invention relates to a vector comprising the nucleic acid molecules of the present invention.

A further aspect of the present invention relates to a host cell comprising a nucleic acid molecule or a vector according to the present invention.

Another aspect of the present invention relates to the use of a protein or a host cell according to the present invention or a lysate or homogenate thereof for reducing D-glucosone to D-fructose.

The use of the xylose reductase enzymes of the present invention having the unique specificities as mentioned above allow to produce compositions having a unique composition since these xylose reductase enzymes are able to produce D-fructose from D-glucosone to a much higher ratio. Hence, a further aspect of the present invention relates to a composition obtainable by a method according to the present invention.

### SHORT DESCRIPTION OF THE FIGURES

Fig. 1 shows a comparison of different xylose reductases to define the GxGxW motif.
Fig. 2 shows possible reduction products, when D-glucosone is used as substrate, D-glucose (via C-2-carbonyl reduction) or D-fructose (via C1-carbonyl reduction).
Fig. 3 shows the D-fructose production process.

### DESCRIPTION OF EMBODIMENTS

The present invention relates to a method for producing D-fructose comprising the step of reducing D-glucosone to D-fructose with a modified xylose reductase enzyme or a functional fragment thereof, wherein the modified xylose reductase enzyme has a lower D-glucose specificity and a lower D-xylose specificity compared to the unmodified xylose reductase enzyme.

The teachings of the present invention allow to modify xylose reductase enzymes to exhibit a lower D-glucose specificity and a lower D-xylose specificity compared to the respective unmodified xylose reductase enzyme. The reduction of both activities results in the formation of D-fructose to a much high extent compared to xylose reductase enzymes having an unmodified D-glucose and/or D-xylose specificity.

As used herein, the term "functional fragment" of a xylose reductase enzyme refers to fragments of xylose reductase enzyme which retain at least 50%, preferably at least 60%, more preferably at least 70%, more preferably at least 80%, more preferably at least 90%, more preferably at least 95%, in particular 100%, of their enzymatic activity in regard to the ability to reduce D-glucosone to D-fructose. "Functional fragments" may comprise truncations of one or more, preferably of 1 to 20 amino acid residues, more preferably of 2 to 15 amino acid residues, at the N-terminal end of the xylose reductase. "Functional fragments" may also comprise truncations of one or more, preferably of 1 to 50 amino acid residues, more preferably of 2 to 25 amino acid residues, at the C-terminal end of the xylose reductase.

As used herein, the term "enzyme specificity" or "specificity of an enzyme" is intended to mean the degree to which an enzyme is able to catalyze a chemical reaction on more than one substrate molecule. An enzyme that can catalyze a reaction on exactly one molecular substrate, but is unable to catalyze a reaction on any other substrate, is said to have very high specificity for its substrate. An enzyme that can catalyze chemical reactions on many substrates is said to have low specificity. In some cases, the specificity of an enzyme is described relative to one or more defined substrates.

Modified xylose reductase enzyme having "lower D-glucose specificity" and "lower D-xylose specificity" means that the modified xylose reductase enzyme catalyzes at least 10%, preferably at least 20%, more preferably at least 30%, more preferably at least 40%, more preferably at least 50%, more preferably at least 60%, more preferably at least 70%, more preferably at least 80%, more preferably at least 90%, more preferably at least 95%, less D-glucose and/or D-xylose compared to the respective unmodified xylose reductase enzyme.

According to a preferred embodiment of the present invention the modified xylose reductase enzyme comprises at least one mutation within the motif GX₁GX₂X₃X₄ present in the unmodified xylose reductase enzyme, X₁ being leucine or phenylalanine, X₂ being leucine or cysteine, X₃ being tryptophan and X₄ being lysine or arginine, wherein X₃ in the modified xylose reductase enzyme is an amino acid residue selected from the group consisting of tyrosine and phenylalanine, preferably phenylalanine.

According to a preferred embodiment of the present invention the protein of the present invention comprises a polypeptide consisting of the GxGxW motif characterized by the alignment shown in Fig. 1.

According to another preferred embodiment of the present invention, the unmodified xylose reductase enzyme of the present invention comprises GxGxW as motif. The tryptophan residue of the motif of the unmodified enzyme can be preferably modified to either tyrosine or phenylalanine resulting in a modified xylose reductase enzyme which significantly prefers D-glucosone over D-glucose and/or D-xylose as substrate.

Xylose reductase comprise the motif GX₁GX₂X₃X₄. It turned out that modifications within this motif result in modified xylose reductase enzymes having a reduced D-glucose and/or D-xylose specificity. The glycine residues at position 1 and 3 of the motif, however, remain unchanged.

Amino acid classification, as used herein, is as follows:
Polar amino acids are serine (Ser, S), threonine (Thr, T), cysteine (Cys, C), glutamine (Gln, Q) and asparagine (Asn, N). Positively charged amino acids are lysine (Lys, K) and arginine (Arg, R) as well as histidine (His, H). Negatively charged amino acids are aspartate (Asp, D) and glutamate (Glu, E) . Non-polar amino acids are glycine (Gly, G), alanine (Ala, A), valine (Val, V), leucine (Leu, L), isoleucine (Ile, I), methionine (Met, M), proline (Pro, P), phenylalanine (Phe, F), tyrosine (Tyr, Y) and tryptophan (Trp, W).

According to a further preferred embodiment of the present invention X₂ and/or X₄ of the modified xylose reductase enzyme differ from X₂ and/or X₄ of the unmodified xylose reductase enzyme, wherein X₂ is selected from the group consisting of threonine, cysteine, valine, leucine, serine, isoleucine, methionine, glycine, proline, alanine, histidine, glutamine and asparagine and/or X₄ is lysine or arginine.

According to a preferred embodiment of the present invention X₂ and/or X₃ of the motif GX₁GX₂X₃X₄ are exchanged in the modified xylose reductase enzyme compared to the unmodified xylose reductase enzyme.

According to a particularly preferred embodiment of the present invention the modified xylose reductase enzyme comprises the motif GX₁GTFK (SEQ ID No. 29).

Modified xylose reductase enzymes comprising motif GX₁GTFK are particularly preferred since such enzymes show a significantly reduced D-glucose and/or D-xylose specificity. Such enzymes can be advantageously used in the conversion of D-glucosone into D-fructose reducing the formation of other reductions products than D-fructose to a minimum.

Xylose reductase enzymes capable to reduce D-glucosone to D-fructose may comprise the motif GYRLFD (SEQ ID No. 30) 15 to 20, preferably 16 to 18, amino acid residues from motif GX₁GX₂X₃X₄ in the C-terminal direction.

According to a preferred embodiment of the present invention said modified xylose reductase enzyme comprises an amino acid sequence having at least 50% sequence identity to SEQ ID No. 1, wherein the amino acid sequence of the modified xylose reductase enzyme, when aligned to the sequence of SEQ ID NO. 1, comprises at least the mutation W24X₃ compared to SEQ ID NO. 1.

SEQ ID No. 1 (KlmXR) (Kluyveromyces marxianus)

MTYLAPTVTLNNGSKMPLVGLGCWKIPNEVCAEQVYEAIKLGYRLFDGAQDYANEKEVGQGI
NRAIKEGIVKREDLVVVSKLWNSFHHPDNVRTAVERTLNDLQLDYLDLFYIHFPLAFKFVPL
DEKYPPGFYTGKDNFAKEIIEEEPVPILDTYRALEKLVDEGLIKSLGISNFSGALIQDLLRG
ARIKPVALQIEHHPYLVQDRLITYAQKVGLQVVAYSSFGPLSFVELNNEKALHTKTLFENDT

IKAIAQKHNVTPSHVLLKWSTQRGIAVIPKSSKKERLLENLKIEETFTLSDEEIKEINGLDQ
GLRFNDPWDWLGNEFPTFI

The terms "sequence identity" and "identity", as used herein, refer to the percentage of identical nucleotide or amino acid matches between at least two nucleotide or amino acid sequences aligned using a standardized algorithm. Such an algorithm can, in a standardized and reproducible manner, insert gaps in the compared sequences to optimize the alignment between two sequences, thus achieving a more meaningful comparison of the two sequences.

Percent identity between sequences may be determined using one or more computer algorithms or programs known in the prior art or described herein. According to the present invention, the Basic Local Alignment Search Tool (BLAST) provided by the National Center for Biotechnology Information (NCBI) (Altschul et al., 1990), is preferably used. The BLAST software series contains several programs, including a tool referred to as "BLAST 2 Sequences", which is used for direct pairwise comparison of two nucleotide or amino acid sequences. "BLAST 2 Sequences" can also be accessed and used interactively via the NCBI World Wide Web page on the Internet. The blastn program (for nucleotide sequences) uses as defaults a word length (W) of 28, an expectation (E) of 0.05, M = 1, N = -2, and a comparison of both strands. For amino acid sequences, the blastp program uses as defaults a word length of 3 and an expectation (E) of 0.05 and the BLOSUM62 scoring matrix (Henikoff & Henikoff, 1989), alignments (B) of 50, expectation (E) of 0.05, M = 1, N = -2.

According to another preferred embodiment of the present invention the amino acid sequence of the modified xylose reductase enzyme when aligned to the sequence of SEQ ID NO. 1, comprises a further mutation C23X₂ compared to SEQ ID NO. 1, wherein X₂ is selected from the group consisting of threonine, cysteine, valine, leucine, serine, isoleucine, methionine, glycine, proline, alanine, histidine, glutamine or asparagine, wherein threonine is particularly preferred.

This second modification allows to further reduce the D-xylose and D-glucose specificity of xylose reductases.

According to a preferred embodiment of the present invention the modified xylose reductase enzyme comprises an amino acid sequence selected from the group consisting of
i) an amino acid sequence having at least 50% sequence identity with any one of SEQ ID No. 1 to 5,
ii) an amino acid sequence encoded by a nucleic acid sequence having an identity to any one of SEQ ID No. 6 to 10 of at least 50%, and
iii) an amino acid sequence encoded by a nucleic acid which binds under stringent conditions to a nucleic acid molecule complementary to any one of nucleic acid sequence SEQ ID No. 6 to 10, wherein stringent conditions comprise washing at 65 °C, and at a salt concentration of 0.1 to 2x SSC (wherein 1x SSC is understood to be a mixture of 0,15 M sodium chloride/0,015 M sodium citrate) .

SEQ ID No. 2 (CgXR) (*Chaetomium globosum*) 
MAPVIKLNSGYDMPQVGFGLWKVDNAVASDVVYNAIKAGYRLFDGACDYGNEVECGQGVARA
ISEGIVKREDLFIVSKLWNTFHDAERVEPIVKKQLADWGIEYFDLYLIHFPVALEWVDPAVR
YPPGWHYDGKEEIRPSKATIQETWTALESLVSKGLSKSIGISNFQAQLIYDLLRYAKIRPAT
LQVEHHPYLVQQELINLAKREGIAVTAYSSFGPASFKEFNMKHADALAPLIEDETIKKIAAK
HNRPASQVLLRWATQRGLAIIPKSTRPQIMAENFQSIDFDLSEEDIATISAFDRGIRFNQPS
NYFPTELLWIFG
SEQ ID No. 3 (CpXR) (*Candida parapsilosis*) 
MSIKLNSGHEMPIVGFGCWKVTNETAADQIYNAIKVGYRLFDGAQDYGNEKEVGEGINRAID
EGLVSRDELFVVSKLWNNYHDPKNVETALNKTLSDLNLEYLDLFLIHFPIAFKFVPIEEKYP
PGFYCGDGDKFHYENVPLLDTWRALESLVQKGKIRSIGISNFNGGLIYDLVRGAKIKPAVLQ
IEHHPYLQQPRLIEFVQSQGIAITGYSSFGPQSFLELESKKALDTPTLFDHETIKSIASKHK
KSSAQVLLRWATQRGIAVIPKSNNPDRLAQNLNVSDFELSKEDLEAINKLDKGLRFNDPWDW
DHIPIFV
SEQ ID No. 4 (CtropXR) (*Candida tropicalis*) 
MSTTVNTPTIKLNSGYEMPLVGFGCWKVTNATAADQIYNAIKTGYRLFDGAEDYGNEKEVGE
GINRAIKDGLVKREELFITSKLWNNFHDPKNVETALNKTLSDLNLDYVDLFLIHFPIAFKFV
PIEEKYPPGFYCGDGDNFHYEDVPLLDTWKALEKLVEAGKIKSIGISNFTGALIYDLIRGAT
IKPAVLQIEHHPYLQQPKLIEYVQKAGIAITGYSSFGPQSFLELESKRALNTPTLFEHETIK
SIADKHGKSPAQVLLRWATQRNIAVIPKSNNPERLAQNLSVVDFDLTKDDLDNIAKLDIGLR
FNDPWDWDNIPIFV
SEQ ID No. 5 (CtenuisXR) (Candida_tenuis) 
MSASIPDIKLSSGHLMPSIGFGCWKLANATAGEQVYQAIKAGYRLFDGAEDYGNEKEVGDGV
KRAIDEGLVKREEIFLTSKLWNNYHDPKNVETALNKTLADLKVDYVDLFLIHFPIAFKFVPI
EEKYPPGFYCGDGNNFVYEDVPILETWKALEKLVAAGKIKSIGVSNFPGALLLDLLRGATIK
PAVLQVEHHPYLQQPKLIEFAQKAGVTITAYSSFGPQSFVEMNQGRALNTPTLFAHDTIKAI
AAKYNKTPAEVLLRWAAQRGIAVIPKSNLPERLVQNRSFNTFDLTKEDFEEIAKLDIGLRFN
DPWDWDNIPIFV
SEQ ID No. 6 (KlmXR) (*Kluyveromyces marxianus*) 
ATGACATACCTCGCACCAACAGTTACCTTGAACAATGGATCCAAGATGCCGCTAGTCGGCTT
GGGATGCTGGAAAATCCCAAACGAAGTGTGTGCCGAACAGGTGTACGAAGCCATCAAGTTGG
GCTACCGCTTGTTCGACGGCGCGCAGGACTACGCCAACGAAAAAGAGGTGGGCCAAGGTATT
AACAGAGCCATCAAGGAAGGAATCGTCAAGAGAGAAGACTTGGTCGTCGTTTCTAAGTTGTG
GAACAGTTTCCACCACCCAGACAACGTGCGTACCGCAGTCGAAAGAACTTTGAACGACTTGC
AATTGGACTACTTGGACTTGTTCTACATCCATTTCCCATTGGCTTTCAAGTTCGTGCCACTA
GACGAGAAGTACCCTCCAGGTTTCTACACAGGTAAGGACAATTTCGCCAAGGAAATCATCGA
AGAGGAGCCTGTCCCAATCTTGGACACCTACAGAGCCCTTGAGAAGTTGGTCGACGAAGGTT
TGATCAAATCTTTGGGTATCTCAAACTTTTCGGGTGCATTGATCCAGGACTTGTTGCGTGGC
GCCCGTATCAAGCCAGTCGCCTTGCAGATCGAACACCACCCATACTTGGTCCAGGACCGCTT
GATCACGTACGCCCAAAAGGTGGGCTTGCAAGTCGTCGCCTACTCCAGTTTCGGCCCACTAT
CCTTTGTCGAGTTGAACAACGAAAAGGCCTTGCACACAAAGACTTTGTTCGAAAACGACACC
ATCAAGGCCATCGCTCAAAAACACAACGTCACCCCATCCCACGTCTTGTTGAAGTGGTCCAC
CCAACGTGGTATCGCCGTCATTCCAAAGTCCTCCAAGAAGGAACGTCTCCTCGAGAACTTGA
AGATCGAAGAGACCTTTACCTTGTCCGACGAAGAGATCAAGGAGATCAACGGCTTGGACCAG
GGATTGAGATTTAACGACCCATGGGACTGGTTGGGCAACGAATTCCCAACCTTTATCTAA
SEQ ID No. 7 (CgXR) (*Chaetomium globosum*) 
atggcgccggtgattaaactgaacagcggctatgatatgccgcaggtgggctttggcctgtg
gaaagtggataacgcggtggcgagcgatgtggtgtataacgcgattaaagcgggctatcgcc
tgtttgatggcgcgtgcgattatggcaacgaagtggaatgcggccagggcgtggcgcgcgcg
attagcgaaggcattgtgaaacgcgaagatctgtttattgtgagcaaactgtggaacacctt
tcatgatgcggaacgcgtggaaccgattgtgaaaaaacagctggcggattggggcattgaat
attttgatctgtatctgattcattttccggtggcgctggaatgggtggatccggcggtgcgc
tatccgccgggctggcattatgatggcaaagaagaaattcgcccgagcaaagcgaccattca
ggaaacctggaccgcgctggaaagcctggtgagcaaaggcctgagcaaaagcattggcatta
gcaactttcaggcgcagctgatttatgatctgctgcgctatgcgaaaattcgcccggcgacc
ctgcaggtggaacatcatccgtatctggtgcagcaggaactgattaacctggcgaaacgcga
aggcattgcggtgaccgcgtatagcagctttggcccggcgagctttaaagaatttaacatga
aacatgcggatgcgctggcgccgctgattgaagatgaaaccattaaaaaaattgcggcgaaa
cataaccgcccggcgagccaggtgctgctgcgctgggcgacccagcgcggcctggcgattat
tccgaaaagcacccgcccgcagattatggcggaaaactttcagagcattgattttgatctga
gcgaagaagatattgcgaccattagcgcgtttgatcgcggcattcgctttaaccagccgagc
aactattttccgaccgaactgctgtggatttttggctaa
SEQ ID No. 8 (CpXR) (*Candida parapsilosis*) 
atgtccattaagctgaatagcggtcacgagatgccaattgtgggttttggttgttggaaggt
gaccaatgaaaccgcggccgatcaaatttataacgcgatcaaggtcggctaccgcttgttcg
acggcgcccaagactacggcaacgagaaagaggttggcgagggcatcaaccgcgcgatcgat
gagggtctggtgtctcgtgacgagctgttcgttgtcagcaagctgtggaataactatcacga
cccgaagaatgtcgaaacggccctgaataaaacgctgagcgatttgaacctggaatacctgg
atctgttcctgatccactttccgatcgcgttcaaatttgtcccgatcgaagagaaataccct
ccgggcttttactgcggcgatggtgataaatttcactatgaaaacgttccgctgctggacac
ctggcgcgccttggaaagcctggtgcagaagggtaaaatccgttccattggtatcagcaatt
tcaacggtggcctgatttacgatctggttcgtggcgcaaagatcaagccggctgtgctgcag
attgagcatcacccgtatctgcaacagcctcgcctgattgagttcgtccagagccagggtat
tgcaatcaccggttattctagcttcggtccacagagcttcctggagttggaaagcaagaaag
cgctggataccccgaccctgttcgaccacgaaacgattaaatccatcgctagcaaacataag
aagagcagcgcacaggtgctgctgcgttgggcgactcaacgtggtattgcggttattccgaa
aagcaacaatccggaccgtctggcacaaaacctgaatgttagcgactttgagttgtcgaaag
aggacctggaagcgatcaacaagctggataaaggtctgcgttttaatgacccgtgggactgg
gatcatattccaatctttgtttaa
SEQ ID. No. 9 (CtropXR) (*Candida tropicalis*) 
atgagcacgaccgttaatactccgacgatcaaactgaactccggttacgagatgccgctggt
tggctttggctgttggaaagttaccaatgcgacggcggaccagatttataacgctatta
aaaccggttaccgtctgttcgatggcgcagaggactacggtaacgagaaagaagtgggtgaa
ggtattaatcgcgcaattaaagacggtctggtcaaacgtgaagagctgtttattacctcgaa
actgtggaataacttccatgatcctaagaacgtggagactgcgctgaataagaccctgagcg
atctgaacctggactatgtggatctgtttctgattcacttcccgattgctttcaaatttgtc
ccgatcgaagaaaagtacccaccgggcttctactgcggtgacggcgacaacttccattatga
ggatgttccgctgctggacacgtggaaggcgctggagaaattggtggaagccggtaagatca
agtccattggcattagcaacttcaccggtgccttgatttacgatttgatccgtggtgcgacc
attaaaccggcggttctgcagatcgagcatcacccgtatctgcaacagccgaaactgatcga
atacgttcagaaagcaggtatcgccatcactggctatagcagcttcggtccacagagcttcc
tggagctggagagcaagcgtgccctgaataccccgacgttgtttgaacacgaaaccatcaag
tctatcgctgacaaacacggtaagagccctgcacaagtcctgctgcgctgggcaacgcaacg
taatattgcggttattccgaagagcaataacccggagcgtctggcgcagaatctgtctgtcg
tcgactttgatttgaccaaggatgacctggataatatcgcgaagctggacatcggcctgcgc
ttcaacgatccgtgggattgggacaacatcccgatctttgtgta
SEQ ID No. 10 (CtenuisXR) (*Candida*_*tenuis*)

ATGAGCGCAAGTATCCCAGACATCAAATTGAGCTCCGGCCACTTAATGCCTTCCATCGGTTT
CGGCTGTTGGAAGCTCGCCAACGCTACCGCCGGTGAACAAGTCTACCAAGCCATCAAGGCCG
GTTACAGATTGTTCGACGGTGCCGAGGACTACGGTAACGAAAAGGAAGTCGGTGACGGTGTC
AAGAGAGCCATCGACGAAGGTCTTGTCAAGAGAGAAGAGATCTTCCTCACCTCCAAGTTGTG
GAACAACTACCACGACCCAAAGAACGTCGAGACCGCCTTGAACAAAACCCTCGCCGACCTTA
AGGTTGACTACGTTGACTTGTTCTTGATCCATTTCCCAATTGCCTTCAAGTTCGTCCCAATC
GAGGAGAAGTACCCACCAGGATTCTACTGTGGTGACGGTAACAACTTCGTCTACGAAGACGT
TCCAATCTTGGAGACCTGGAAGGCCCTCGAGAAGTTGGTTGCTGCCGGTAAGATTAAGTCCA
TCGGTGTCTCTAACTTCCCAGGTGCTTTGCTCTTGGACTTGCTCAGAGGTGCTACCATCAAG
CCAGCTGTCTTGCAAGTTGAGCACCACCCATACTTGCAACAACCAAAGTTGATCGAGTTCGC
TCAAAAGGCCGGTGTTACCATCACCGCCTACTCTTCTTTCGGTCCTCAATCTTTCGTTGAGA
TGAACCAAGGTAGAGCTTTGAACACCCCAACCTTGTTCGCACACGACACCATCAAGGCTATT
GCTGCCAAGTACAACAAGACCCCAGCTGAGGTTTTATTGAGATGGGCCGCCCAAAGAGGTAT
CGCTGTCATTCCAAAGTCTAACCTCCCAGAGAGATTAGTCCAAAACAGAAGTTTCAACACCT
TCGACTTGACCAAGGAGGACTTCGAGGAAATCGCCAAGTTGGACATCGGCTTGAGATTCAAC
GACCCATGGGACTGGGACAACATTCCAATCTTCGTTTAA

Alternatively, the xylose reductase enzymes of the present invention preferably comprise an amino acid sequence encoded by a nucleic acid that binds under stringent conditions to a nucleic acid molecule complementary to the nucleic acid sequence SEQ ID No. 6 to 10. As used herein, stringent conditions refer to conditions under which so-called specific hybrids, but not nonspecific hybrids, are formed.

Hybridization may be performed by conventionally known procedures, such as those described in J. Sambrook et al, Molecular Cloning, A Laboratory Manual, 2nd Ed, Cold Spring Harbor Laboratory (1989). The stringent condition is a condition in which washing is performed at 65 °C, and at a salt concentration of 0.1 to 2x SSC (wherein 1xSSC is understood to be a mixture of 0,15 M sodium chloride/0,015 M sodium citrate).

The xylose reductase enzyme of the present invention can be modified once, twice, three, four or five times by a water-soluble polymer. A water-soluble polymer is polyethylene glycol, for example. The binding of the polyethylene glycol preferably takes place at the N-terminal end of the protein according to the present invention. The protein of the present invention can also be bound to a solid body such as polyethylene, polystyrene, polysaccharide, cellulose or cellulose derivatives.

The xylose reductase enzyme of the present invention, preferably obtained by recombinant expression in E. coli, can be subjected to heat precipitation of the E. coli background enzymes in order to reach after ultrafiltration and DNA-removal food enzyme compliance. For example, the heat incubation may be performed at, e. g., 45 °C for, but not limited to, 30 min.

Furthermore, the modified xylose reductase enzyme of the present invention may be part of a fusion protein. Such a fusion protein may comprise next to the enzyme of the present invention at least a further polypeptide at the N-terminal and/or C-terminal end. Fusion proteins can, for example, be separated more easily from other proteins or are expressed in the cells in larger amounts.

According to a preferred embodiment of the present invention the modified xylose reductase enzyme comprises an amino acid sequence encoded by a nucleic acid sequence having an identity to SEQ ID No. 1 of at least 60%, preferably of at least 70%, more preferably of at least 80%, more preferably of at least 90%, in particular of at least 95%.

According to a further preferred embodiment of the present invention the modified xylose reductase enzyme comprises or consists of an amino acid sequence selected from the group consisting of SEQ ID No. 11 to 28.
SEQ ID No. 11 (CtropXR W26Y) (*Candida tropicalis*) 
MSTTVNTPTIKLNSGYEMPLVGFGCYKVTNATAADQIYNAIKTGYRLFDGAEDYGNEKEVGE
GINRAIKDGLVKREELFITSKLWNNFHDPKNVETALNKTLSDLNLDYVDLFLIHFPIAFKFV
PIEEKYPPGFYCGDGDNFHYEDVPLLDTWKALEKLVEAGKIKSIGISNFTGALIYDLIRGAT
IKPAVLQIEHHPYLQQPKLIEYVQKAGIAITGYSSFGPQSFLELESKRALNTPTLFEHETIK
SIADKHGKSPAQVLLRWATQRNIAVIPKSNNPERLAQNLSVVDFDLTKDDLDNIAKLDIGLR
FNDPWDWDNIPIFV
SEQ ID No. 12 (KlmXR W24Y) (*Kluyveromyces marxianus*) 
MTYLAPTVTLNNGSKMPLVGLGCYKIPNEVCAEQVYEAIKLGYRLFDGAQDYANEKEVGQGI
NRAIKEGIVKREDLVVVSKLWNSFHHPDNVRTAVERTLNDLQLDYLDLFYIHFPLAFKFVPL
DEKYPPGFYTGKDNFAKEIIEEEPVPILDTYRALEKLVDEGLIKSLGISNFSGALIQDLLRG
ARIKPVALQIEHHPYLVQDRLITYAQKVGLQVVAYSSFGPLSFVELNNEKALHTKTLFENDT
IKAIAQKHNVTPSHVLLKWSTQRGIAVIPKSSKKERLLENLKIEETFTLSDEEIKEINGLDQ
GLRFNDPWDWLGNEFPTFI
SEQ ID No. 13 (KlmXR C23T W24F) (*Kluyveromyces marxianus*) 
MTYLAPTVTLNNGSKMPLVGLGTFKIPNEVCAEQVYEAIKLGYRLFDGAQDYANEKEVGQGI
NRAIKEGIVKREDLVVVSKLWNSFHHPDNVRTAVERTLNDLQLDYLDLFYIHFPLAFKFVPL
DEKYPPGFYTGKDNFAKEIIEEEPVPILDTYRALEKLVDEGLIKSLGISNFSGALIQDLLRG
ARIKPVALQIEHHPYLVQDRLITYAQKVGLQVVAYSSFGPLSFVELNNEKALHTKTLFENDT
IKAIAQKHNVTPSHVLLKWSTQRGIAVIPKSSKKERLLENLKIEETFTLSDEEIKEINGLDQ
GLRFNDPWDWLGNEFPTFI
SEQ ID No. 14 (CgXR W21Y) (*Chaetomium globosum*) 
MAPVIKLNSGYDMPQVGFGLYKVDNAVASDVVYNAIKAGYRLFDGACDYGNEVECGQGVARA
ISEGIVKREDLFIVSKLWNTFHDAERVEPIVKKQLADWGIEYFDLYLIHFPVALEWVDPAVR
 
YPPGWHYDGKEEIRPSKATIQETWTALESLVSKGLSKSIGISNFQAQLIYDLLRYAKIRPAT
LQVEHHPYLVQQELINLAKREGIAVTAYSSFGPASFKEFNMKHADALAPLIEDETIKKIAAK
HNRPASQVLLRWATQRGLAIIPKSTRPQIMAENFQSIDFDLSEEDIATISAFDRGIRFNQPS
NYFPTELLWIFG
SEQ ID No. 15 (CpXR C18T W19F) (*Candida parapsilosis*) 
MSIKLNSGHEMPIVGFGTFKVTNETAADQIYNAIKVGYRLFDGAQDYGNEKEVGEGINRAID
EGLVSRDELFVVSKLWNNYHDPKNVETALNKTLSDLNLEYLDLFLIHFPIAFKFVPIEEKYP
PGFYCGDGDKFHYENVPLLDTWRALESLVQKGKIRSIGISNFNGGLIYDLVRGAKIKPAVLQ
IEHHPYLQQPRLIEFVQSQGIAITGYSSFGPQSFLELESKKALDTPTLFDHETIKSIASKHK
KSSAQVLLRWATQRGIAVIPKSNNPDRLAQNLNVSDFELSKEDLEAINKLDKGLRFNDPWDW
DHIPIFV
SEQ ID No. 16(KlmXR C23T W24F K25R) *(Kluyveromyces marxianus)* 
MTYLAPTVTLNNGSKMPLVGLGTFRIPNEVCAEQVYEAIKLGYRLFDGAQDYANEKEVGQGI
NRAIKEGIVKREDLVVVSKLWNSFHHPDNVRTAVERTLNDLQLDYLDLFYIHFPLAFKFVPL
DEKYPPGFYTGKDNFAKEIIEEEPVPILDTYRALEKLVDEGLIKSLGISNFSGALIQDLLRG
ARIKPVALQIEHHPYLVQDRLITYAQKVGLQVVAYSSFGPLSFVELNNEKALHTKTLFENDT
IKAIAQKHNVTPSHVLLKWSTQRGIAVIPKSSKKERLLENLKIEETFTLSDEEIKEINGLDQ
GLRFNDPWDWLGNEFPTFI
SEQ ID No. 17 (KlmXR C23V W24F) *(Kluyveromyces marxianus)* 
MTYLAPTVTLNNGSKMPLVGLGVFKIPNEVCAEQVYEAIKLGYRLFDGAQDYANEKEVGQGI
NRAIKEGIVKREDLVVVSKLWNSFHHPDNVRTAVERTLNDLQLDYLDLFYIHFPLAFKFVPL
DEKYPPGFYTGKDNFAKEIIEEEPVPILDTYRALEKLVDEGLIKSLGISNFSGALIQDLLRG
ARIKPVALQIEHHPYLVQDRLITYAQKVGLQVVAYSSFGPLSFVELNNEKALHTKTLFENDT
IKAIAQKHNVTPSHVLLKWSTQRGIAVIPKSSKKERLLENLKIEETFTLSDEEIKEINGLDQ
GLRFNDPWDWLGNEFPTFI
SEQ ID No. 18 (KlmXR C23L W24F) *(Kluyveromyces marxianus)* 
MTYLAPTVTLNNGSKMPLVGLGLFKIPNEVCAEQVYEAIKLGYRLFDGAQDYANEKEVGQGI
NRAIKEGIVKREDLVVVSKLWNSFHHPDNVRTAVERTLNDLQLDYLDLFYIHFPLAFKFVPL
DEKYPPGFYTGKDNFAKEIIEEEPVPILDTYRALEKLVDEGLIKSLGISNFSGALIQDLLRG
ARIKPVALQIEHHPYLVQDRLITYAQKVGLQVVAYSSFGPLSFVELNNEKALHTKTLFENDT
 
IKAIAQKHNVTPSHVLLKWSTQRGIAVIPKSSKKERLLENLKIEETFTLSDEEIKEINGLDQ
GLRFNDPWDWLGNEFPTFI
SEQ ID No. 19 (KlmXR C23S W24F) (*Kluyveromyces marxianus*) 
MTYLAPTVTLNNGSKMPLVGLGSFKIPNEVCAEQVYEAIKLGYRLFDGAQDYANEKEVGQGI
NRAIKEGIVKREDLVVVSKLWNSFHHPDNVRTAVERTLNDLQLDYLDLFYIHFPLAFKFVPL
DEKYPPGFYTGKDNFAKEIIEEEPVPILDTYRALEKLVDEGLIKSLGISNFSGALIQDLLRG
ARIKPVALQIEHHPYLVQDRLITYAQKVGLQVVAYSSFGPLSFVELNNEKALHTKTLFENDT
IKAIAQKHNVTPSHVLLKWSTQRGIAVIPKSSKKERLLENLKIEETFTLSDEEIKEINGLDQ
GLRFNDPWDWLGNEFPTFI
SEQ ID No. 20 (KlmXR C23I W24F) (*Kluyveromyces marxianus*) 
MTYLAPTVTLNNGSKMPLVGLGIFKIPNEVCAEQVYEAIKLGYRLFDGAQDYANEKEVGQGI
NRAIKEGIVKREDLVVVSKLWNSFHHPDNVRTAVERTLNDLQLDYLDLFYIHFPLAFKFVPL
DEKYPPGFYTGKDNFAKEIIEEEPVPILDTYRALEKLVDEGLIKSLGISNFSGALIQDLLRG
ARIKPVALQIEHHPYLVQDRLITYAQKVGLQVVAYSSFGPLSFVELNNEKALHTKTLFENDT
IKAIAQKHNVTPSHVLLKWSTQRGIAVIPKSSKKERLLENLKIEETFTLSDEEIKEINGLDQ
GLRFNDPWDWLGNEFPTFI
SEQ ID No. 21 (KlmXR C23M W24F) (*Kluyveromyces marxianus*) 
MTYLAPTVTLNNGSKMPLVGLGMFKIPNEVCAEQVYEAIKLGYRLFDGAQDYANEKEVGQGI
NRAIKEGIVKREDLVVVSKLWNSFHHPDNVRTAVERTLNDLQLDYLDLFYIHFPLAFKFVPL
DEKYPPGFYTGKDNFAKEIIEEEPVPILDTYRALEKLVDEGLIKSLGISNFSGALIQDLLRG
ARIKPVALQIEHHPYLVQDRLITYAQKVGLQVVAYSSFGPLSFVELNNEKALHTKTLFENDT
IKAIAQKHNVTPSHVLLKWSTQRGIAVIPKSSKKERLLENLKIEETFTLSDEEIKEINGLDQ
GLRFNDPWDWLGNEFPTFI
SEQ ID No. 22 (KlmXR W24F) *(Kluyveromyces marxianus)* 
MTYLAPTVTLNNGSKMPLVGLGCFKIPNEVCAEQVYEAIKLGYRLFDGAQDYANEKEVGQGI
NRAIKEGIVKREDLVVVSKLWNSFHHPDNVRTAVERTLNDLQLDYLDLFYIHFPLAFKFVPL
DEKYPPGFYTGKDNFAKEIIEEEPVPILDTYRALEKLVDEGLIKSLGISNFSGALIQDLLRG
ARIKPVALQIEHHPYLVQDRLITYAQKVGLQVVAYSSFGPLSFVELNNEKALHTKTLFENDT
IKAIAQKHNVTPSHVLLKWSTQRGIAVIPKSSKKERLLENLKIEETFTLSDEEIKEINGLDQ
GLRFNDPWDWLGNEFPTFI
SEQ ID No. 23 (KlmXR C23G W24F) (*Kluyveromyces marxianus*) 
MTYLAPTVTLNNGSKMPLVGLGGFKIPNEVCAEQVYEAIKLGYRLFDGAQDYANEKEVGQGI
NRAIKEGIVKREDLVVVSKLWNSFHHPDNVRTAVERTLNDLQLDYLDLFYIHFPLAFKFVPL
DEKYPPGFYTGKDNFAKEIIEEEPVPILDTYRALEKLVDEGLIKSLGISNFSGALIQDLLRG
ARIKPVALQIEHHPYLVQDRLITYAQKVGLQVVAYSSFGPLSFVELNNEKALHTKTLFENDT
IKAIAQKHNVTPSHVLLKWSTQRGIAVIPKSSKKERLLENLKIEETFTLSDEEIKEINGLDQ
GLRFNDPWDWLGNEFPTFI
SEQ ID No. 24 (KlmXR C23P W24F) (*Kluyveromyces marxianus*) 
MTYLAPTVTLNNGSKMPLVGLGPFKIPNEVCAEQVYEAIKLGYRLFDGAQDYANEKEVGQGI
NRAIKEGIVKREDLVVVSKLWNSFHHPDNVRTAVERTLNDLQLDYLDLFYIHFPLAFKFVPL
DEKYPPGFYTGKDNFAKEIIEEEPVPILDTYRALEKLVDEGLIKSLGISNFSGALIQDLLRG
ARIKPVALQIEHHPYLVQDRLITYAQKVGLQVVAYSSFGPLSFVELNNEKALHTKTLFENDT
IKAIAQKHNVTPSHVLLKWSTQRGIAVIPKSSKKERLLENLKIEETFTLSDEEIKEINGLDQ
GLRFNDPWDWLGNEFPTFI
SEQ ID No. 25 (KlmXR C23A W24F) (*Kluyveromyces marxianus*) 
MTYLAPTVTLNNGSKMPLVGLGAFKIPNEVCAEQVYEAIKLGYRLFDGAQDYANEKEVGQGI
NRAIKEGIVKREDLVVVSKLWNSFHHPDNVRTAVERTLNDLQLDYLDLFYIHFPLAFKFVPL
DEKYPPGFYTGKDNFAKEIIEEEPVPILDTYRALEKLVDEGLIKSLGISNFSGALIQDLLRG
ARIKPVALQIEHHPYLVQDRLITYAQKVGLQVVAYSSFGPLSFVELNNEKALHTKTLFENDT
IKAIAQKHNVTPSHVLLKWSTQRGIAVIPKSSKKERLLENLKIEETFTLSDEEIKEINGLDQ
GLRFNDPWDWLGNEFPTFI
SEQ ID No. 26 (KlmXR C23H W24F) *(Kluyveromyces marxianus)* 
MTYLAPTVTLNNGSKMPLVGLGHFKIPNEVCAEQVYEAIKLGYRLFDGAQDYANEKEVGQGI
NRAIKEGIVKREDLVVVSKLWNSFHHPDNVRTAVERTLNDLQLDYLDLFYIHFPLAFKFVPL
DEKYPPGFYTGKDNFAKEIIEEEPVPILDTYRALEKLVDEGLIKSLGISNFSGALIQDLLRG
ARIKPVALQIEHHPYLVQDRLITYAQKVGLQVVAYSSFGPLSFVELNNEKALHTKTLFENDT
IKAIAQKHNVTPSHVLLKWSTQRGIAVIPKSSKKERLLENLKIEETFTLSDEEIKEINGLDQ
GLRFNDPWDWLGNEFPTFI
SEQ ID No. 27 (KlmXR C23Q W24F) *(Kluyveromyces marxianus)* 
MTYLAPTVTLNNGSKMPLVGLGQFKIPNEVCAEQVYEAIKLGYRLFDGAQDYANEKEVGQGI
NRAIKEGIVKREDLVVVSKLWNSFHHPDNVRTAVERTLNDLQLDYLDLFYIHFPLAFKFVPL
DEKYPPGFYTGKDNFAKEIIEEEPVPILDTYRALEKLVDEGLIKSLGISNFSGALIQDLLRG
ARIKPVALQIEHHPYLVQDRLITYAQKVGLQVVAYSSFGPLSFVELNNEKALHTKTLFENDT
IKAIAQKHNVTPSHVLLKWSTQRGIAVIPKSSKKERLLENLKIEETFTLSDEEIKEINGLDQ
GLRFNDPWDWLGNEFPTFI
SEQ ID No. 28 (KlmXR C23N W24F) (*Kluyveromyces marxianus*)

MTYLAPTVTLNNGSKMPLVGLGNFKIPNEVCAEQVYEAIKLGYRLFDGAQDYANEKEVGQGI
NRAIKEGIVKREDLVVVSKLWNSFHHPDNVRTAVERTLNDLQLDYLDLFYIHFPLAFKFVPL
DEKYPPGFYTGKDNFAKEIIEEEPVPILDTYRALEKLVDEGLIKSLGISNFSGALIQDLLRG
ARIKPVALQIEHHPYLVQDRLITYAQKVGLQVVAYSSFGPLSFVELNNEKALHTKTLFENDT
IKAIAQKHNVTPSHVLLKWSTQRGIAVIPKSSKKERLLENLKIEETFTLSDEEIKEINGLDQ
GLRFNDPWDWLGNEFPTFI

According to a preferred embodiment of the present invention the D-glucosone is obtainable by oxidizing enzymatically D-glucose, preferably by using a pyranose-2-oxidase.

In the method of the present invention D-glucosone is reduced to D-fructose using the modified xylose reductase enzyme of the present invention. D-glucosone can be provided from any available source. However, it turned out to be advantageous that D-glucosone is used which is obtainable by oxidizing enzymatically D-glucose, preferably by using a pyranose-2-oxidase. This allows to use any source of D-glucose to produce D-fructose.

D-glucose present in the reaction mixture does not have any influence on the reduction reaction of D-glucosone to D-fructose since the modified xylose reductase enzyme of the present invention has a reduced D-glucose specificity. Due to this property, it is possible to reduce almost or even all of the D-glucosone present in the reaction mixture into D-fructose.

Due to the specificity of the modified xylose reductase of the present invention D-glucose of any source can be used for producing D-glucosone. Suitable sources of D-glucose in a method according to the present invention are, for example, enzymatic or non-enzymatic hydrolysates of starch, in particular corn starch, enzymatic or non-enzymatic hydrolysates of saccharose or enzymatic or non-enzymatic hydrolysates of cellulose. Cellulose which can be used in a method according to the present invention may be obtained, for example, from a biomass, preferably from a lignocellulosic biomass such as, e.g, wood, straw such as wheat straw, corn straw, bagasse, sisal, energy grasses. For example, amylases may be used for the enzymatic hydrolysis of corn starch. For example, invertases are suitable for the enzymatic cleavage of saccharose. For example, cellulases may be used for the enzymatic cleavage of cellulose. An acid-catalyzed cleavage, for example, is suitable for the non-enzymatic cleavage of said multiple sugars.

Pyranose-2-oxidase, for instance, used to oxidize D-glucose to form D-glucosone requires oxygen. Said oxygen can be introduced to the reaction mixture as known in the art and can be made available, for example, through contact with ambient air or an increased oxygen supply, for example by compressed air or the injection of pure oxygen.

It is preferred to use an overpressure of air/oxygen during performing the method of the present invention, preferably when pyranose-2-oxidase is additionally employed. "Overpressure", as used herein, means more than ambient pressure, preferably more than 1.1 bar, more preferably more than 1.2 bar, more preferably more than 1.5 bar, more preferably more than 2 bar, more preferably more than 5 bar. The upper limit of the overpressure does preferably not exceed 20 bar, preferably 10 bar, more preferably 5 bar.

According to a preferred embodiment of the present invention nascent H₂O₂ is removed with a catalase.

During the reaction of the oxidase (e.g. pyranose-2-oxidase), H₂O₂ is formed which is preferably removed from the reaction mixture. The removal of H₂O₂ may occur according to conventional methods and preferably occurs enzymatically, for example, with the aid of a catalase. For example, a catalase is added to the reaction mixture. Suitable catalases are known and are obtainable, for example, from *Aspergillus* sp., *Corynebacterium glutamicum* or from bovine liver.

The oxidation reaction to form D-glucosone and the reduction reaction to form D-fructose are carried out preferably in the same reaction batch without intermediates being isolated, in particular wherein two enzymatic redox reactions involved in the product formation and an enzymatic system for cofactor regeneration are performed in one reaction batch without isolating an intermediate, is herein referred to as a "one-pot synthesis". In the process, either all the involved enzymes can be added simultaneously, or at first a portion of the enzymes is added, for example, the enzyme (s) for step (a) and, with a time delay, another portion of the enzymes, for example, the enzyme(s) for step (b). Before the second portion of the enzymes is added, the enzymes which are already present in the reaction batch may, for example, be inactivated, for instance, by a conventional method such as, e.g., an increase in the temperature, for example, to 65 °C for 10 min.

According to another preferred embodiment of the present invention during the reduction of D-glucosone to D-fructose and/or during the oxidation of D-glucose to D-glucosone NAD(P)H/NAD(P)⁺ is added to the reaction mixture.

According to a further embodiment of the present invention NAD(P)H/ NAD(P)⁺ is recycled by a suitable cofactor regeneration system that requires the presence of a suitable cosubstrate which is used up during the regeneration of the redox cofactors. Cosub-strates which can be used, for example, are alcohols such as, e.g., isopropyl alcohol (2-propanol, IPA), lactic acid and salts thereof, oxygen, hydrogen and/or formic acid and salts thereof.

For the regeneration of redox cofactors, a redox enzyme is used. Redox enzymes which come into consideration if redox cofactors NAD(P)H/NAD(P)⁺ are used, include, for example, dehydrogenases, e. g., alcohol dehydrogenases, lactacte dehydrogenases, formate dehydrogenases, preferably alcohol dehydrogenases. Methods for regenerating cofactors as outlined above are disclosed, for instance, in US 2015/353978.

At the beginning of the conversion of D-glucose to D-fructose, which can be carried out with or without overpressure - as outlined above - and may involve the aforementioned enzymes like alcohol dehydrogenases, the IPA level or the level of other alcohols may be 5 to 15% (v/v), preferably 8 to 12 % (v/v), more preferably approx. 10% (v/v), if IPA or other alcohols are used as cosubstrate. As the process progresses, the IPA level, for instance, drops, and acetone concomitantly accumulates. It is noteworthy that the xylose reductase of the present invention surprisingly tolerates the organic solvents throughout the entire process, which typically results in a conversion >99% fructose at the end of the operation. A further advantage is that the concentration of at least 5% organic solvent (mixture of isopropanol and acetone) in the product solution suppresses microbial growth.

Another aspect of the present invention relates to a modified xylose reductase enzyme or a functional fragment thereof, wherein the modified xylose reductase enzyme has a lower D-glucose specificity and a lower D-xylose specificity compared to the unmodified xylose reductase enzyme.

A further aspect of the present invention relates to a nucleic acid molecule encoding a modified xylose reductase enzyme according to the present invention. The nucleic acid molecule may be a DNA or an RNA molecule.

A further aspect of the present invention relates to a vector comprising a nucleic acid molecule according to the present invention.

The term "vector", as used herein, refers to a nucleic acid molecule capable of transporting another nucleic acid to which it is linked. Useful vectors are those capable of autonomous replication and/or expression of the nucleic acid linked to it. Vectors capable of directing the expression of genes to which they are operatively linked are referred to herein as "expression vectors". Expression vectors used in recombinant DNA technology often have the form of a "plasmid", which usually refers to a circular double-stranded DNA loop that does not bind to a chromosome when in the form of a vector. As used herein, "plasmid" and "vector" are used interchangeably because plasmids are the most commonly used form of vector. However, other forms of expression vectors that perform equivalent functions and are subsequently known in the art are also included.

Suitable cloning vectors for overexpression and production of the proteins of the present invention include, for example, pKK223-3, pTrc99a, pUC, pTZ, pSK, pBluescript, pGEM, pQE, pET, PHUB, pPLc, pKC30, pRM1/pRM9, pTrxFus, pAS1, pGEx, pMAL or pTrx.

The nucleic acid molecule of the present invention encoding the protein of the present invention may be operably linked to a promoter. The term "operably linked", as used herein, means that a selected nucleotide sequence (such as encoding a protein described herein) is in close proximity to the promoter to allow the promoter to regulate the expression of the selected nucleic acid molecule. In addition, the promoter is located upstream of the selected nucleotide sequence in the direction of transcription and translation. By "operably linked" is meant that when an appropriate molecule (such as a transcriptional activator protein) is bound to a regulatory sequence, the nucleotide sequence and the regulatory sequence are linked in such a way that the nucleic acid molecule/gene is expressed.

Suitable expression promoters include, for example, trp-lac (tac)-promoter, trp-lac (trc) promoter, lac-promoter, T5-promoter, T7-promoter and λpL-promoter.

Another aspect of the present invention relates to a host cell comprising a nucleic acid molecule or a vector according to the present invention.

Host cells comprising a nucleic acid molecule or a vector according to the present invention can be used for producing the modified xylose reductase enzyme of the present invention. Suitable host cells include bacteria like *Escherichia coli* and yeast cells like *Komagataella phaffii.*

A further aspect of the present invention relates to the use of a modified xylose reductase enzyme or a host cell according to the present invention or a cell suspension, a lysate or a homogenate or immobilized or lyophilized or spray-dried.

The modified xylose reductase enzyme of the present invention can be used in the method of the present invention either as whole cells, cell homogenate, cell lysate, in a completely (at least 95%, preferably at least 98%) or partially purified state, or as a functional fragment thereof. The method of the present invention is carried out with the protein according to the invention or with cells containing the protein according to the invention. In doing so, the cells used can be provided in a native, permeabilized or lysed state.

The present invention is further illustrated by the following embodiments and examples, however, without being restricted thereto.

### EMBODIMENTS

1. A method for producing D-fructose comprising the step of reducing D-glucosone to D-fructose with a modified xylose reductase enzyme or a functional fragment thereof, wherein the modified xylose reductase enzyme has a lower D-glucose specificity and a lower D-xylose specificity compared to the unmodified xylose reductase enzyme.
2. The method of embodiment 1, wherein the modified xylose reductase enzyme comprises at least one mutation within the motif GX₁GX₂X₃X₄ present in the unmodified xylose reductase enzyme, X₁ being leucine or phenylalanine, X₂ being leucine or cysteine, X₃ being tryptophan and X₄ being lysine or arginine, wherein X₃ in the modified xylose reductase enzyme is an amino acid residue selected from the group consisting of tyrosine and phenylalanine, preferably phenylalanine.
3. The method of embodiment 2, wherein X₂ and/or X₄ of the modified xylose reductase enzyme differ from X₂ and/or X₄ of the unmodified xylose reductase enzyme, wherein X₂ is selected from the group consisting of threonine, cysteine, valine, leucine, serine, isoleucine, methionine, glycine, proline, alanine, histidine, glutamine and asparagine and/or X₄ is lysine or arginine.
4. The method of any one of embodiments 1 to 3, wherein the modified xylose reductase enzyme comprises the motif GX₁GTFK.
5. The method of any one of embodiments 1 to 4, wherein the modified xylose reductase enzyme comprises further the motif GYRLFD.
6. The method of any one of embodiments 1 to 5, wherein said modified xylose reductase enzyme comprises an amino acid sequence having at least 50% sequence identity to SEQ ID No. 1, wherein the amino acid sequence of the modified xylose reductase enzyme, when aligned to the sequence of SEQ ID NO. 1, comprises at least the mutation W24X₃ compared to SEQ ID NO. 1.
7. The method of embodiment 6, wherein the amino acid sequence of the modified xylose reductase enzyme when aligned to the sequence of SEQ ID NO. 1, comprises a further mutation C23X₂ compared to SEQ ID NO. 1, wherein X₂ is selected from the group consisting of threonine, cysteine, valine, leucine, serine, isoleucine, methionine, glycine, proline, alanine, histidine, glutamine or asparagine.
8. The method of any one of embodiments 1 to 7, wherein the modified xylose reductase enzyme comprises an amino acid sequence selected from the group consisting of
   i) an amino acid sequence having at least 50% sequence identity with any one of SEQ ID No. 1 to 5,
   ii) an amino acid sequence encoded by a nucleic acid sequence having an identity to any one of SEQ ID No. 6 to 10 of at least 50%, and
   iii) an amino acid sequence encoded by a nucleic acid which binds under stringent conditions to a nucleic acid molecule complementary to any one of nucleic acid sequence SEQ ID No. 6 to 10, wherein stringent conditions comprise washing at 65 °C, and at a salt concentration of 0.1 to 2x SSC.
9. The method of any one of embodiments 1 to 8, wherein the modified xylose reductase enzyme comprises an amino acid sequence encoded by a nucleic acid sequence having an identity to SEQ ID No. 1 of at least 60%, preferably of at least 70%, more preferably of at least 80%, more preferably of at least 90%, in particular of at least 95%.
10. The method of any one of embodiments 1 to 9, wherein the modified xylose reductase enzyme comprises or consists of an amino acid sequence selected from the group consisting of SEQ ID No. 11 to 29.
11. The method of any one of embodiments 1 to 10, wherein the D-glucosone is obtainable by oxidizing D-glucose enzymatically, preferably by using a pyranose-2-oxidase.
12. The method of embodiment 11, wherein nascent H₂O₂ is removed with a catalase.
13. The method of any one of embodiments 1 to 12, wherein during the reduction of D-glucosone to D-fructose and/or during the oxidation of D-glucose to D-glucosone NAD(P)H/NAD(P)⁺ is added to the reaction mixture.
14. The method of embodiment 13, wherein NAD(P)H/ NAD(P)⁺ is recycled by a suitable cofactor regeneration system, and wherein the cofactor regeneration system includes consuming a cosubstrate.
15. The method of embodiment 14, wherein the cosubstrate is selected from an alcohol, lactic acid and salts thereof, pyruvic acid and salts thereof, oxygen, hydrogen and / or formic acid and salts thereof.
16. A modified xylose reductase enzyme or a functional fragment thereof, wherein the modified xylose reductase enzyme has a lower D-glucose specificity and a lower D-xylose specificity compared to the unmodified xylose reductase enzyme.
17. The modified xylose reductase enzyme of embodiment 16, wherein the modified xylose reductase enzyme comprises at least one mutation within the motif GX₁GX₂X₃X₄ present in the unmodified xylose reductase enzyme, X₁ being leucine or phenylalanine, X₂ being leucine or cysteine, X₃ being tryptophan and X₄ being lysine or arginine, wherein X₃ in the modified xylose reductase enzyme is an amino acid residue selected from the group consisting of tyrosine and phenylalanine, preferably phenylalanine.
18. The modified xylose reductase enzyme of embodiment 17, wherein X₂ and/or X₄ of the modified xylose reductase enzyme differ from X₂ and/or X₄ of the unmodified xylose reductase enzyme, wherein X₂ is selected from the group consisting of threonine, cysteine, valine, leucine, serine, isoleucine, methionine, glycine, proline, alanine, histidine, glutamine and asparagine and/or X₄ is lysine or arginine.
19. The modified xylose reductase enzyme of any one of embodiments 16 to 18, wherein the modified xylose reductase enzyme comprises the motif GX₁GTFK.
20. The modified xylose reductase enzyme of any one of embodiments 16 to 19, wherein the modified xylose reductase enzyme comprises further the motif GYRLFD.
21. The modified xylose reductase enzyme of any one of embodiments 16 to 20, wherein said modified xylose reductase enzyme comprises an amino acid sequence having at least 50% sequence identity to SEQ ID No. 1, wherein the amino acid sequence of the modified xylose reductase enzyme, when aligned to the sequence of SEQ ID NO. 1, comprises at least the mutation W24X₃ compared to SEQ ID NO. 1.
22. The modified xylose reductase enzyme of embodiment 21, wherein the amino acid sequence of the modified xylose reductase enzyme when aligned to the sequence of SEQ ID NO. 1, comprises a further mutation C23X₂ compared to SEQ ID NO. 1, wherein X₂ is selected from the group consisting of threonine, cysteine, valine, leucine, serine, isoleucine, methionine, glycine, proline, alanine, histidine, glutamine or asparagine.
23. The modified xylose reductase enzyme of any one of embodiments 16 to 22, wherein the modified xylose reductase enzyme comprises an amino acid sequence selected from the group consisting of
   i) an amino acid sequence having at least 50% sequence identity with any one of SEQ ID No. 1 to 5,
   ii) an amino acid sequence encoded by a nucleic acid sequence having an identity to any one of SEQ ID No. 6 to 10 of at least 50%, and
   iii) an amino acid sequence encoded by a nucleic acid which binds under stringent conditions to a nucleic acid molecule complementary to any one of nucleic acid sequence SEQ ID No. 6 to 10, wherein stringent conditions comprise washing at 65 °C, and at a salt concentration of 0.1 to 2x SSC.
24. The modified xylose reductase enzyme of any one of embodiments 16 to 23, wherein the modified xylose reductase enzyme comprises an amino acid sequence encoded by a nucleic acid sequence having an identity to any one of SEQ ID No. 6 to 10 of at least 60%, preferably of at least 70%, more preferably of at least 80%, more preferably of at least 90%, in particular of at least 95%.
25. The modified xylose reductase enzyme of any one of embodiments 16 to 24, wherein the modified xylose reductase enzyme comprises or consists of an amino acid sequence selected from the group consisting of SEQ ID No. 11 to 28.
26. A nucleic acid molecule encoding a modified xylose reductase enzyme according to any one of any one of embodiments 16 to 25.
27. A vector comprising a nucleic acid molecule according to any one of embodiments 26.
28. A host cell comprising a nucleic acid molecule according to embodiment 26 or a vector according to embodiment 27.
29. Use of a modified xylose reductase enzyme according to any one of embodiments 16 to 25 or a host cell according to embodiment 28 or a lysate or homogenate thereof, heat-incubated or not, for reducing D-glucosone to D-fructose.
30. Composition obtainable by a method according to any one of embodiments 1 to 15.

### EXAMPLES

### Materials

D-fructose, NADPH tetrasodium salt, and NADP⁺ were obtained from PanReac AppliChem (ITW Reagents). D-glucose, and IPTG (isopropyl-β-D-thiogalactopyranoside) were obtained from Sigma-Al-drich. D-glucosone was prepared in house, and triethanolamine (TEA) was purchased from Chem-Lab NV.

### Production of the enzymes & production of the lysates

### General information on the expression of recombinant enzymes in E. coli.

For recombinant enzyme production in an *Escherichia coli* strain, the gene to be expressed was first amplified in a PCR using the genomic DNA or its equivalent synthetically adapted to the codon usage of *E. coli* as a template together with specific oligonucleotides additionally carrying recognition sequences for restriction endonucleases and isolated from the reaction mixture. After nucleic acid digestion with restriction enzymes SphI and HindIII, the gene fragment encoding the target enzyme was ligated into the backbone of the expression vector pQE70-Kan cut with SphI and HindIII. The ligation product was transformed into chemically competent *E. coli* cells Top10F and the resulting colonies are used for plasmid isolation and restriction analysis. The result of the cloning step was verified by restriction enzyme digestion and DNA sequencing. The resulting construct carries the target gene under the IPTG-inducible T5 promoter.

For overexpression of the enzyme in E. coli, the resulting expression plasmid was transformed into the competent expression cells RB791. After 24 h incubation at 37 °C, resulting colonies were inoculated into LB medium for expression assays.

The next day, expression cultures were inoculated with an optical density OD₅₅₀ of 0.02 and shaken at 37 °C until an OD₅₅₀ of 0.3 was reached. The temperature was then lowered to 25 °C and the cultures were induced with 0.5 mM IPTG when an OD₅₅₀ of 0.5 was reached. After 22 h, the cultures were harvested (separated from the medium by centrifugation in the form of a cell pellet) and analyzed for expression of the recombinant enzyme by SDS gel electrophoresis and an activity assay (use in use assay or optical enzymatic assay).

### Production of cell lysates by GEA disruption

To prepare a cell suspension (volume > 50 ml), the cell pellet prepared according to the above procedure was weighed into a suitable vessel and dissolved in buffer (100 mM triethanolamine (TEA-HCl) pH 7). The mass fraction of biomass is usually 20%, with the remainder accounted for by the buffer.
A GEA Lab Homogenizer PandaPLUS 2000 was used for cell disruption (pressure about 1000 bar).

The homogenates were centrifuged for 10 min at 4 °C and 16000 rpm (Hermle Z 446 K centrifuge) to separate the insoluble cell fragments and obtain the lysate.

### Kinetic measurements

Activity measurements with enzyme preparations were performed as follows:
A defined volume (10 µl) of enzyme dilution was mixed in a cuvette of a total volume of one mL consisting of 0.2 mM cofactor (NADH and NADPH, respectively) and 100 mM TEA-HCl pH7,0. The change of absorbance (ΔA) measured at 340 nm is a measure of enzyme activity according to Lambert-Beer's law (ΔA = ε·Ac·d; where ε is 6.22 l/(mmol·cm) for NAD(P)H; Δc is the change in concentration and d is the optical path length of the cuvette (1 cm). One enzyme unit U corresponds to the amount of enzyme that catalyzes the conversion of one µmol substrate per minute under defined conditions. The activity given below are expressed as U/g biomass (wet weight). Substrate concentrations used were - if not otherwise stated - D-glucose, 200 mM; D-glucosone, 20 mM;
D-fructose, 200 mM.
PCR-mediated Mutagenesis
XR mutations were generated using standard overlap extension PCR (for example described by Hilgarth and Lanigan (2019).

### High Performance Liquid Chromatography (HPLC)

HPLC (high performance liquid chromatography) was used to quantify D-fructose, D-glucose, D-glucosone and D-sorbitol. Detection was by means of a refractive index detector. A Phenomenex Rezex RCM-Monosaccharide Ca2+ column (temperature: 80 °C) column with a corresponding pre-column was used for the measurement and eluted with 3.5% isopropanol (flow rate: 0.5 ml/min).

### Example 1

### Kinetic characteristics of XRs (optical-enzymatic assay)

**Table 1. Results of kinetic measurements.**

| | **D-xylose [U/g]** | **D-glucosone [U/g]** | **D-glucose [U/g]** |
|---|---|---|---|
| **Klm WT** | 1717 | 1850 | 203 |
| **Klm W24Y** | 42 | 810 | 9 |
| **Klm C23T W24F** | 118 | 1904 | 5 |
| **Ctrop WT** | 2830 | 3408 | 412 |
| **Ctrop W26Y** | 69 | 957 | 5 |
| **Ctrop C25T W26F** | 108 | 1923 | 6 |
| **Cg WT** | 5439 | 1939 | 373 |
| **Cg W21Y** | 26 | 327 | 6 |
| **Cp WT** | 1117 | 1174 | 196 |
| **Cp C18T W19F** | 44 | 1115 | 3 |

This example clearly demonstrates that the mutated XRs preferentially use D-glucosone as substrate.

### Example 2

Different sugars (D-fructose, 50 g/L, or D-glucose, 50 g/L, or D-glucosone, 60 g/L) were incubated together with alcohol dehydrogenase (ADH) (*Methanobacterium veterum;* GenBank accession no. MCZ3365290) (5.5 U) in absence or presence of different xylose reductases (XRs) (1.0 U, based on D-glucosone activity or the same biomass was used, when D-glucose or D-fructose served as substrate) in an aqueous TEA-HCl buffer (50 mM, pH 8.0) containing 15 µM NADP⁺. The reaction was carried out at 30° C under continuous shaking (1200 rpm) for 16 h in a final volume of 500 µl containing a final isopropanol concentration of 10%. During the reaction the ADH-mediated cofactor recycling system consumes isopropanol to regenerate the cofactor and produce acetone.

In this example, the redox cofactor NADP⁺ is recycled via ADH/isopropanol to form acetone and NADPH+H⁺. The recycled cofactor NADPH+H⁺ may be used by XR to perform the reduction. There are different products possible when D-glucosone is used as substrate, D-glucose after C-2-carbonyl reduction or D-fructose via C1-carbonyl reduction (Fig. 2).

**Table 2. Products detected after XR-mediated reduction of different substrates.**

| **substrate** | **ADH** | **XR** | **D-glucose [%]** | **D-glucosone [%]** | **D-fructose [%]** | **D-sorbitol [%]** |
|---|---|---|---|---|---|---|
| **D-fructose** | - | - | / | / | 100.0 | / |
| | + | - | / | / | 100.0 | / |
| | + | **Klm WT** | / | / | 100.0 | / |
| | + | **Klm W24Y** | / | / | 100.0 | / |
| | + | **Klm C23T W24F** | / | / | 100.0 | / |
| **D-glucose** | - | - | 100.0 | / | / | / |
| | + | - | 100.0 | / | / | / |
| | + | **Klm WT** | 65.2 | / | / | 34.8 |
| | + | **Klm W24Y** | 98.1 | / | / | 1.9 |
| | + | **Klm C23T W24F** | 98.3 | / | / | 1.7 |
| **D-glucosone** | - | - | / | 100 | / | / |
| | + | - | / | 100 | / | / |
| | + | **Klm WT** | <LOD | 46.9 | 51.5 | / |
| | + | **Klm W24Y** | <LOD | <LOQ | 100.0 | / |
| | + | **Klm C23T W24F** | <LOD | 13.8 | 85.0 | / |

| | | | | | | |
|---|---|---|---|---|---|---|
| **<LOD - below level of detection** **<LOQ - below level of quantification** | | | | | | |

### Results

Neither Klm WT nor the GxGxW motif mutants Klm W24Y and Klm C23T W24F generated D-sorbitol from D-fructose. Moreover, D-glucose reduction was drastically reduced when the GxGxW motif mutants Klm W24Y and Klm CW(23,24)TF) were tested compared to Klm WT. When D-glucosone was used as substrate, the XR mutants of the GxGxW motif (Klm W24Y and Klm C23T W24F) surprisingly generated D-fructose suggesting that the mutants catalyze aldehyde reduction (C1-carbonyl group) and not ketone reduction (C2-carbonyl group) in D-glucosone. This finding is in disagreement with the data published by Kratzer et al. (2006) who showed that the tryptophan residue in the GXGXW motif (defined in Figure 1 of Kratzer et al. (2006)) leads to aldehyde reduction (C1-carbonyl group), whereas mutation to Y or F results in a strong preference for ketone reduction (C2-carbonyl group) .

Thus, it has been shown that GxGxW motif mutants show significantly altered substrate specificity towards D-glucosone without changing the catalytic mechanism.

### Example 3

### Kinetic characteristics of screened XR mutants (optical-enzymatic assay)

**Table 3. Results of kinetic measurements of screened XRs**

| | **D-xylose [%WT]** | **D-glucosone [%C23T W24F]** | **D-glucose [%WT]** |
|---|---|---|---|
| Klm WT | 100 | 97 | 100 |
| **Klm W24Y** | 2 | 43 | 4 |
| **Klm C23T W24F** | 7 | 100 | 2 |
| **Klm C23T W24F K25R** | 6 | **80** | 1 |
| **Klm C23V W24F** | 8 | **86** | 1 |
| **Klm C23L W24F** | 6 | **70** | 1 |
| **Klm C23S W24F** | 6 | **80** | 1 |
| **Klm C23I W24F** | 5 | **109** | 0 |
| **Klm C23M W24F** | 6 | **75** | 0 |
| **Klm W24F** | 5 | **110** | 0 |
| **Klm C23G W24F** | 2 | **52** | 0 |
| **Klm C23P W24F** | 0 | **32** | 0 |
| **Klm C23A W24F** | 17 | **86** | 0 |
| **Klm C23H W24F** | 10 | **28** | 0 |
| **Klm C23Q W24F** | 4 | **44** | 0 |
| **Klm C23N W24F** | 4 | **91** | 0 |

This example clearly demonstrates that the mutated XRs (marked in bold) preferentially use D-glucosone as substrate and have similar activities as Klm W24Y and Klm C23T W24F. Also note that Klm C23T W24F K25R has similar substrate preference as Klm C23T W24F.

### Example 4

### D-fructose production from invert sugar with mutated XRs

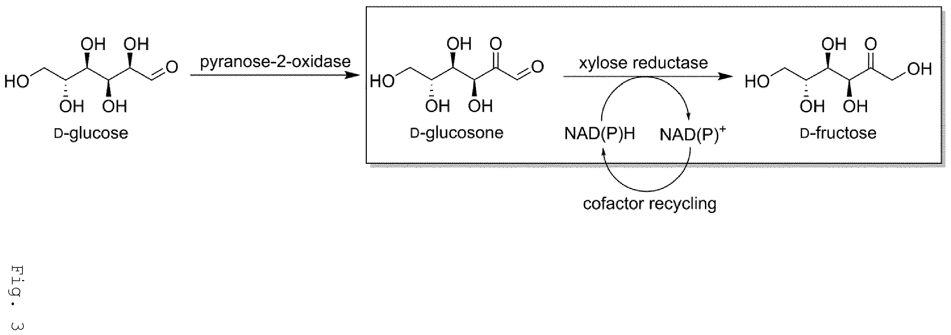

A D-glucose/D-fructose mixture (51% D-glucose, 49% D-fructose) (total final sugar concentration, 200-300 g/L as stated and Table 4) was dissolved together with pyranose-2-oxidase (*Trametes versicolor;* uniprot accession no. P79076) (1.6 U), catalase (*Micrococcus luteus;* uniprot accession no. P29422) (1600 U), different xylose reductases (XR)s (2.7 U) and alcohol dehydrogenase (ADH) (*Methanobacterium veterum;* GenBank accession no. MCZ3365290) (30 U), in an aqueous TEA-HCl buffer (50 mM, pH 8.0). The reaction was carried out at 30° C under continuous shaking (1200 rpm) for 24 h in a total volume of 500 µl containing 50 µl isopropanol (final 10%). During the reaction the ADH-mediated cofactor recycling system consumes isopropanol to regenerate the cofactor and produce volatile acetone (Fig. 3).

In this example, the redox cofactor NADP⁺ is recycled via ADH/isopropanol to form NADPH+H⁺ and volatile acetone. XR uses the recycled cofactor NADPH+H⁺ to mediate the D-glucosone reduction to form D-fructose and NADP⁺.

**Table 4. Results of the D-fructose production experiments.**

| **Sugar Cone. [g/L]** | **XR (2.7 U)** | **D-glucose [%]** | **D-glucosone [%]** | **D-fructose [%]** | **D-sorbitol [%]** | **Conversion [%]** |
|---|---|---|---|---|---|---|
| **200** | **Klm WT** | / | 26.9 | 73.1 | / | 47 |
| **300** | **Klm W24Y** | / | / | 100 | / | 100 |
| **300** | **Klm C23T W24F** | / | / | 100 | / | 100 |
| **200** | **Ctrop WT** | / | 33.8 | 66.2 | / | 34 |
| **300** | **Ctrop W2 6Y** | / | 2.7 | 97.3 | / | 95 |
| **200** | **Cg WT** | 0.4 | 7.0 | 86.6 | 2.0 | 74 |
| **300** | **Cg W21Y** | / | 0.6 | 98.9 | 0.4 | 98 |
| **200** | **Cp WT** | / | 33.7 | 66.3 | / | 34 |
| **300** | **Cp C18T W19F** | / | 2.7 | 97.6 | 0.3 | 95 |

This example clearly demonstrates that the mutated XRs preferentially use D-glucosone as substrate during D-fructose production without byproduct formation from D-glucose.

### Example 5

### XR preparation via heat incubation

20% GEA Lysate (as described in methods section) was subjected to heat incubation (45 °C, 30 min, 300 UpM) in the Eppendorf thermomixerC and centrifuged (10 min; 20,000 g; 4 °C). Cleared lysate (termed "heat-incubated") and control lysate before heat incubation (termed "control") were characterized by kinetic measurements using 100 mM D-glucosone (instead of 20 mM) as substrate **(Table 5).**

**Table 5. Kinetic characterization of heat-incubated XRs.**

| **Enzyme** | **Sample** | **D-glucosone [U/g]** | **% of control** |
|---|---|---|---|
| **Klm W24Y** | **Control** | 3414 | 100 |
| | **Heat-incubated** | 3186 | 93 |
| **Klm C23T W24F** | **Control** | 3639 | 100 |
| | **Heat-incubated** | 3299 | 91 |

This example clearly demonstrates that enzyme preparation including heat incubation did not affect D-glucosone activity significantly.

### LITERATURE

Milić, M., de Maria, P. D., & Kara, S. (2023). A patent survey on the biotechnological production of 2,5-furandicarboxylic acid (FDCA): Current trends and challenges. EFB Bioeconomy Journal, 100050. https://doi.org/10.1016/j.bioeco.2023.100050
Grembecka, M. (2015). Sugar alcohols-their role in the modern world of sweeteners: a review. European Food Research and Technology, 241, 1-14.https://doi.org/10.1007/s00217-015-2437-7
Karmali A, Coelho J. Bioconversion of D-glucose into D-glucosone by glucose 2-oxidase from Coriolus versicolor at moderate pressures. Appl Biochem Biotechnol. 2011 Apr;163(7) :906-17. doi: 10.1007/s12010-010-9094-x.
Kratzer, R., Leitgeb, S., Wilson, D. K., & Nidetzky, B. (2006). Probing the substrate binding site of Candida tenuis xylose reductase (AKR2B5) with site-directed mutagenesis. Biochemical Journal, 393(1), 51-58. https://doi.org/10.1042/BJ20050831
Liang, L., Zhang, J., & Lin, Z. (2007). Altering coenzyme specificity of Pichia stipitis xylose reductase by the semi-rational approach CASTing. Microbial Cell Factories, 6, 36. https://doi.org/10.1186/1475-2859-6-36
Neuhauser, W., Haltrich, D., Kulbe, K. D., & Nidetzky, B. (1997). NAD(P)H-dependent aldose reductase from the xylose-assimilating yeast Candida tenuis. Isolation, characterization and biochemical properties of the enzyme. Biochemical Journal, 326(3), 683-92. https://doi.org/10.1042/bj3260683
Hilgarth, R.S., & Lanigan T. M. (2019). Optimization of overlap extension PCR for efficient transgene construction. Meth-odsX, 7, 100759. https://doi.org/10.1016/j.mex.2019.12.001
Henikoff, S., & Henikoff, J. G. (1992). Amino acid substitution matrices from protein blocks. Proceedings of the National Academy of Sciences of the United States of America, 89(22), 10915-10919. https://doi.org/10.1073/pnas.89.22.10915
Sambrook, J., Fritsch, E. R., & Maniatis, T. (1989). Molecular Cloning: A Laboratory Manual (2nd ed.). Cold Spring Harbor, NY: Cold Spring Harbor Laboratory Press.
Altschul, S. F., Gish, W., Miller, W., Myers, E. W., & Lipman, D. J. (1990). Basic local alignment search tool. Journal of Molecular Biology, 215(3), 403-410. https://doi.org/10.1016/S0022-2836(05)80360-2

## Claims

1. A method for producing D-fructose comprising the step of reducing D-glucosone to D-fructose with a modified xylose reductase enzyme or a functional fragment thereof, wherein the modified xylose reductase enzyme has a lower D-glucose specificity and a lower D-xylose specificity compared to the unmodified xylose reductase enzyme.

2. The method of claim 1, wherein the modified xylose reductase enzyme comprises at least one mutation within the motif GX₁GX₂X₃X₄ present in the unmodified xylose reductase enzyme, X₁ being leucine or phenylalanine, X₂ being leucine or cysteine, X₃ being tryptophan and X₄ being lysine or arginine, wherein X₃ in the modified xylose reductase enzyme is an amino acid residue selected from the group consisting of tyrosine and phenylalanine, preferably phenylalanine.

3. The method of claim 2, wherein X₂ and/or X₄ of the modified xylose reductase enzyme differ from X₂ and/or X₄ of the unmodified xylose reductase enzyme, wherein X₂ is selected from the group consisting of threonine, cysteine, valine, leucine, serine, isoleucine, methionine, glycine, proline, alanine, histidine, glutamine and asparagine and/or X₄ is lysine or arginine.

4. The method of any one of claims 1 to 3, wherein the modified xylose reductase enzyme comprises the motif GX₁GTFK.

5. The method of any one of claims 1 to 4, wherein the modified xylose reductase enzyme comprises further the motif GYRLFD.

6. The method of any one of claims 1 to 5, wherein said modified xylose reductase enzyme comprises an amino acid sequence having at least 50% sequence identity to SEQ ID No. 1, wherein the amino acid sequence of the modified xylose reductase enzyme, when aligned to the sequence of SEQ ID NO. 1, comprises at least the mutation W24X₃ compared to SEQ ID NO. 1.

7. The method of claim 6, wherein the amino acid sequence of the modified xylose reductase enzyme when aligned to the sequence of SEQ ID NO. 1, comprises a further mutation C23X₂ compared to SEQ ID NO. 1, wherein X₂ is selected from the group consisting of threonine, cysteine, valine, leucine, serine, isoleucine, methionine, glycine, proline, alanine, histidine, glutamine or asparagine.

8. The method of any one of claims 1 to 7, wherein the modified xylose reductase enzyme comprises an amino acid sequence encoded by a nucleic acid sequence having an identity to SEQ ID No. 1 of at least 60%, preferably of at least 70%, more preferably of at least 80%, more preferably of at least 90%, in particular of at least 95%.

9. The method of any one of claims 1 to 8, wherein during the reduction of D-glucosone to D-fructose and/or during the oxidation of D-glucose to D-glucosone NAD(P)H/NAD(P)⁺ is added to the reaction mixture.

10. A modified xylose reductase enzyme or a functional fragment thereof, wherein the modified xylose reductase enzyme has a lower D-glucose specificity and a lower D-xylose specificity compared to the unmodified xylose reductase enzyme.

11. The modified xylose reductase enzyme of claim 10, wherein the modified xylose reductase enzyme comprises at least one mutation within the motif GX₁GX₂X₃X₄ present in the unmodified xylose reductase enzyme, X₁ being leucine or phenylalanine, X₂ being leucine or cysteine, X₃ being tryptophan and X₄ being lysine or arginine, wherein X₃ in the modified xylose reductase enzyme is an amino acid residue selected from the group consisting of tyrosine and phenylalanine, preferably phenylalanine.

12. The modified xylose reductase enzyme of claim 11, wherein X₂ and/or X₄ of the modified xylose reductase enzyme differ from X₂ and/or X₄ of the unmodified xylose reductase enzyme, wherein X₂ is selected from the group consisting of threonine, cysteine, valine, leucine, serine, isoleucine, methionine, glycine, proline, alanine, histidine, glutamine and asparagine and/or X₄ is lysine or arginine.

13. The modified xylose reductase enzyme of any one of claims 10 to 12, wherein the modified xylose reductase enzyme comprises the motif GX₁GTFK.

14. A nucleic acid molecule encoding a modified xylose reductase enzyme according to any one of any one of claims 10 to 13.

15. A host cell comprising a nucleic acid molecule according to claim 14.

16. Use of a modified xylose reductase enzyme according to any one of claims 10 to 13 or a host cell according to claim 15 or a lysate or homogenate thereof, heat-incubated or not, for reducing D-glucosone to D-fructose.

17. Composition obtainable by a method according to any one of claims 1 to 9.
